# EUROPEAN PATENT SPECIFICATION

(11) **EP 2 879 726 B1**
(45) Date of publication and mention of the grant of the patent: **21.10.2015**
(21) Application number: 13744439.4
(22) Date of filing: 29.07.2013
(51) Int. Cl.: A61L 27/12, A61L 27/36, A61L 27/50, A61L 27/54

(54) **HYDROPHILIC PHOSPHATE GROUP CONTAINING DEHYDRATED PARTIALLY PURIFIED BONE REPLACEMENT MATERIAL**
HYDROPHILES PHOSPHATGRUPPENHALTIGES DEHYDRIERTES, TEILWEISE GEREINIGTES KNOCHENERSATZMATERIAL
GROUPE PHOSPHATE HYDROPHILE CONTENANT UN MATÉRIAU DE SUBSTITUTION OSSEUSE PURIFIÉ ET PARTIELLEMENT DÉSHYDRATÉ

(30) Priority: 31.07.2012 EP 12005559; 31.07.2012 EP 12005560
(43) Date of publication of application: 10.06.2015
(73) Proprietor: Geistlich Pharma AG, 6110 Wolhusen (CH)
(72) Inventor: IMHOF, Cornel, 6005 Luzern (CH); SCHLÖSSER, Lothar, 64297 Darmstadt (DE); SCHÄFER, Birgit, 6170 Schüpfheim (CH); BUFLER, Michael, 6205 Eich (CH)
(74) Representative: Savatier, Yves
(86) International application number: PCT/EP2013/002240
(87) International publication number: WO 2014/019672

(56) References cited:
- US-A1- 2010 030 340
- US-A1- 2010 166 879
- US-B1- 6 293 970

## Description

### Field of the invention

The present invention relates to a hydrophilic dehydrated partially purified bone replacement material of natural origin having a defined isotonic solution suction capacity and containing a phosphate group apt to act as precursor of hydroxyapatite and a process for preparing that hydrophilic dehydrated partially purified bone replacement material.

### Background of the invention

A bone defect site may result from infection, disease, trauma, developmental malformation, malignancy, surgery or other factors. Often bone volume of a certain size needs to be generated, for instance to augment a diseased jaw. In such cases a solid graft is preferably used which can additionally be stabilized. Filling materials in particulate form are limited to small cavities as healing naturally emerges only as long as no or very limited movement occurs between a distracted segment and the surrounding bone.

Several techniques, such as the use of filling materials or grafts of natural or synthetic origin, to replace diseased or missing parts are known. Due to its natural architecture, bone is very porous. In general, bone replacement material tries to mimic the chemistry and microstructure of human bone. Certain physico-chemical parameters such as crystallinity, solubility, particle size, porosity, pore structure and pore size of the material are of pivotal importance and may greatly influence bone compatibility and bone integration. An inappropriate combination of those parameters leads to failure of bone repair. It is known that pore sizes influence cell in-growth and proliferation, vascularisation, and/or cell-adhesion processes. In view of these findings, it can be emphasized that bone replacement material of natural origin has some important advantages over synthetic materials. Although autogenous bone is widely considered to be the gold standard, its application has several shortcomings and both, autogenic and allogenic bones are available only in limited quantities.

In recent years, a great deal of attention has been focussed on the preservation of the biomechanical properties of animal bone while, in parallel, satisfying the stringent regulatory biological and chemical-physical requirements for the development of bone replacement materials of animal origin.

A commercially available bone replacement material is TUTOBONE®, which is a bovine cancellous bone block (available from Tutogen Medical). It is a dehydrated, partially purified bone transplant in which the native mechanical strength is largely maintained.

U.S. Patent No. 6,942,961 describes to a two-step method for dehydrating biological tissues for producing transplants with a preserved bone structure such as TUTOBONE®. In a first step, the tissue is partially dehydrated with an organic, water-miscible solvent. In a second step, the tissue is further dehydrated by freeze drying.

TUTOBONE®, like other commercially available dehydrated partially purified bone replacement materials of animal origin with a preserved bone structure and mechanical strength, does not provide satisfying hydrophilicity and bone integration characteristics. In order to overcome the low hydrophilicity of commercially available bone blocks of animal origin, the surgeon has to rinse the bone blocks with physiological saline during or before the surgery. Such bone blocks have to be wetted by placing them for example in an empty syringe and rinsed several times with physiological saline. This is time-consuming and not practical in a surgery or hospital setting. Besides, it cannot be guaranteed, that such treated bone blocks are completely wetted and do not contain air bubbles. Such air bubbles may prevent a rapid integration of the bone replacement material. At the air-to-blood interface, no growth or regeneration is possible. The direct structural and functional connection between the surrounding living tissue and the bone replacement material is insufficient. Blood, blood components, and the cells of the patient need to integrate the graft material rapidly after implantation.

Thus, there is a need for a dehydrated partially purified bone replacement material of animal origin with a preserved bone structure and mechanical strength that has sufficient hydrophilicity to be easily wetted and rehydrated.

U.S. Patent N0. 6,293,970 and US2010/0030340 disclose a plasticized load-bearing bone graft comprising a cleaned (i.e. partially purified) non-demineralized load-bearing bone graft which is impregnated with one or more plasticizers, the plasticizers being notably saccharides such as glucose, sucrose and D-galactose, and C₂ to C₇ polyols such as glycerol, adonitol, sorbitol, ribitol, galactitol, mannitol and xylitol, and the bone graft being "any bone or piece thereof obtained from a donor, for example a human or animal and/or cadaver donor". The term "plasticized" here means that free and loosely bound waters of hydration in the bone tissue are replaced with a plasticizer without altering the orientation of the collagen fibers and the associated mineral phase, thereby preserving the structure and mechanical properties of natural bone. The plasticizer is taught to be used for stabilizing the bone graft and thus prevent fractures or microfractures developing or propagating during storage, during implantation or after implantation, and for preventing the risk of disease transmission: There is no mention of using the plasticizer as a wetting agent, the issue of hydrophilicity of the bone graft being not at all dealt with in those documents. In U.S. Patent No. 6,293,970 the plasticizer is disclosed to be used at 3 % to 30 % by w/w of bone and to allow the bone graft to be directly implanted into a patient following only a brief washing in sterile isotonic saline to remove the "plasticizer associated with the immediate surfaces of the grafts" or following an extended washing (about one hour) with isotonic saline to remove "as much plasticizer as possible" (see in particular column 8, lines 14-62 and column 12, lines 10-27). In US2010/0030340, the plasticizer is disclosed to be used in "minimal quantities", which here means the minimum amount to replace the waters of hydration in the bone tissue, using a plasticizing composition containing more than 70 % (v/v) plasticizer, with the option of either implanting the plasticized graft without rehydration, or quickly rinsing the implant in sterile isotonic saline prior to implantation (see in particular [0035] and [0126]), thereby as in US Patent No. 6293970 removing only the plasticizer associated with the external surfaces of the graft.

In the above US patent documents, the plasticizer replaces a natural water of hydration without altering the orientation of the collagen fibers and the associated mineral phase: It is thus embedded in the structure of the bone graft and therefore difficult to eliminate by washing with sterile isotonic saline prior to implantation. The effective amount of plasticizer that remains in the plasticized partially purified material after a quick rinsing with isotonic saline is difficult to evaluate: Probably, when the plasticizer is not in excess with regard to the replaced natural water of hydration, most of the plasticizer present remains in the plasticized partially purified material. In vivo the plasticizer will be only slowly washed away by physiological fluids.

Possible patient non tolerance of the bone replacement material, a foreign body introduced in his body, is always a potential problem in clinical implantation, especially if the bone replacement material contains a large amount of an additive such as a plasticizer or wetting agent which is a non physiological substance.

An objective of the present invention is to provide a hydrophilic dehydrated partially purified bone replacement material of natural origin apt to be easily wetted and hydrated, which does not have the drawbacks of the bone replacement materials of the prior art, notably those of U.S. Patent No. 6,942,961 (TUTOBONE®) and those of U.S. Patent Nos. 6,293,970 and US2010/0030340, and which presents improved new bone formation capacity within the scaffold of the bone replacement material.

That objective is attained by the invention as defined in the appended claims.

### Summary of the Invention

The present invention provides a hydrophilic dehydrated partially purified bone replacement material of natural origin, wherein substantially all non-collagenous organic material is removed while inorganic, porous osseous structure and collagenous structure of natural bone are substantially preserved, characterized in that the bone replacement material contains 0.05 to 1.5 w/w % of at least one of a saccharide or a sugar alcohol, and 0.7 to 5.6 w/w % of a phosphate group selected from the group consisting of phosphate HPO₄²⁻ and H₂PO₄⁻, this phosphate group being part of a physiologically acceptable salt which is sodium phosphate buffer.

This invention also provides a process for preparing the hydrophilic dehydrated partially purified bone replacement material according to the embodiment above comprising the steps of: (a) providing a partially purified bone replacement material of natural origin by first extracting lipids with organic solvents, there treating the delipidized material with a solution containing a chaotropic agent and carrying out extensive washing with water, (b) soaking the dehydrated partially purified bone replacement material of natural origin in a solution containing 0.05-0.85 w/w % of at least one of a saccharide or a sugar alcohol, thereby providing hydrophilicity to the partially purified bone replacement material, and 10-1000 mM of a phosphate group selected from the group consisting of phosphate HPO₄²⁻ and H₂PO₄⁻, this phosphate group being part of sodium phosphate buffer, (c) freeze-drying the soaked hydrophilic partially purified bone replacement material, thereby performing dehydration, and (d) sterilizing the hydrophilic partially purified bone freeze-dried bone replacement material.

The invention also provides a use of 0.7 to 5.6 w/w % of a phosphate group a phosphate group apt to react with the calcium ions of physiological fluids to give a precursor of hydroxyapatite selected from the group consisting of phosphate HPO₄²⁻ and H₂PO₄⁻, this phosphate group being part of sodium phosphate buffer, for enhancing the new bone formation capacity of a partially purified bone replacement material of natural origin from which substantially all non-collagenous organic material is removed while inorganic, porous osseous structure and collagenous structure of natural bone are substantially preserved, which contains 0.05 to 1.5 w/w % of at least one of a saccharide or a sugar alcohol.

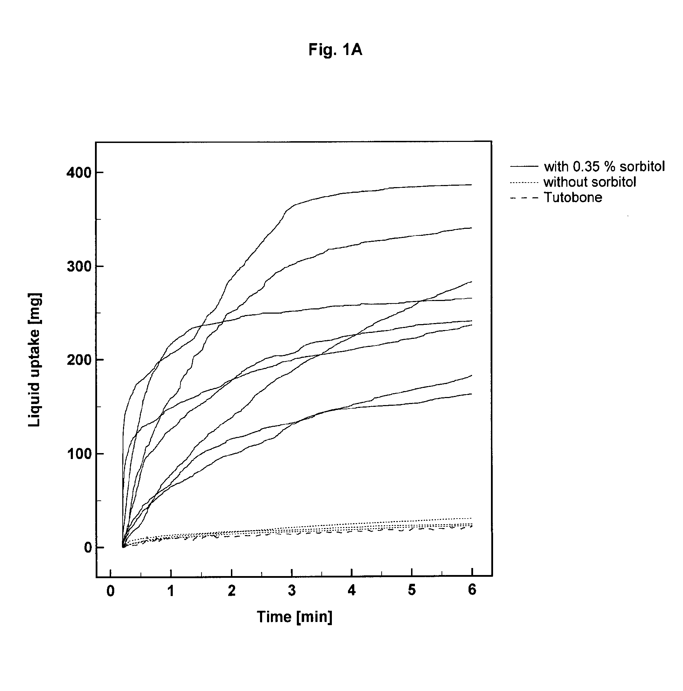

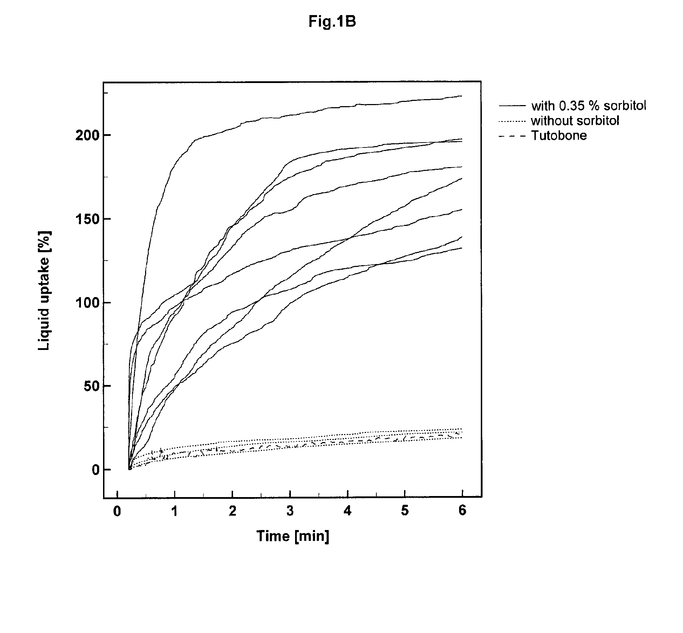

As shown by an isotonic solution suction test (see notably Example 3 and Figures 1A and 1B), the dehydrated partially purified bone replacement material of the invention is hydrophilic and thus apt to be immediately integrated into the surrounding tissue without the risk of forming air bubbles. Thanks to its low content of a saccharide or a sugar alcohol, a non-physiological substance, it will minimally perturb the functioning of the human body and thus be optimally tolerated by the patient.

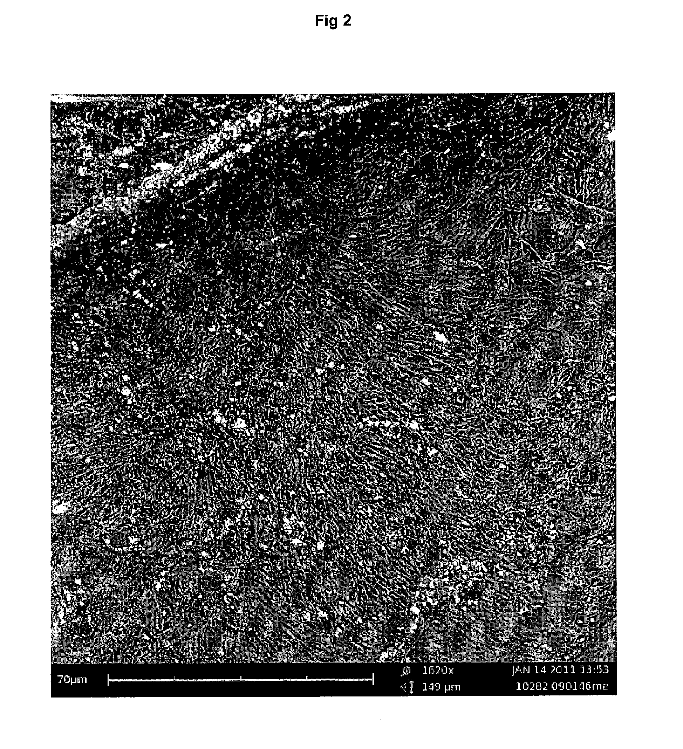

As shown by in vitro experiments of incubating a block of the dehydrated partially purified bone replacement material of the invention in a medium similar to physiological fluids containing calcium ions (see notably Example 4 and Figure 2), the phosphate group released reacts with the calcium ions to form a white precipitate of calcium phosphates on collagen fibrils of the block, these calcium phosphates being precursors of hydroxyapatite. This shows that in vivo, as in natural systems (see S. Gajjeraman et al. 2007 The Journal of Biological Chemistry 282, 2, pp. 1193-1204 and S. Bodhak et al. 2009 Acta Biomaterialia 5, pp. 2178-2188), the nucleation and growth of bone mineral products will take place on the collagen matrix of the hydrophilic dehydrated partially purified bone replacement material, the phosphate group playing a role in those processes.

The phosphate group thus plays an important role in promoting bone formation. Upon implantation in various animals, notably rats and dogs, the hydrophilic dehydrated partially purified bone replacement material of the invention shows excellent new bone formation capacity with no or very few adverse histopathological events (inflammatory or foreign body reactions).

### Brief description of drawings

Figure 1A shows a comparison of the isotonic solution suction capacity (liquid uptake in mg) in correlation with time for 8 different blocks of a hydrophilic dehydrated partially purified bone replacement material of equine origin containing 0.35 % w/w sorbitol and 1.4 % w/w of (HPO₄²⁻ and H₂PO₄⁻) according to the present invention, 3 different blocks of a dehydrated partially purified bone replacement material of natural origin according to the prior art, and commercially available bovine bone substitution material TUTOBONE® (Tutogen Medical GmbH).
Figure 1B shows the relative isotonic solution suction capacity (w/w ratio between the liquid uptake and the dehydrated block expressed in %) in correlation with time for 8 different blocks of a hydrophilic dehydrated partially purified bone replacement material of equine origin containing 0.35 % w/w sorbitol and 1.4 % w/w of (HPO₄²⁻ and H₂PO₄⁻) according to the present invention, compared to 3 different blocks of a dehydrated partially purified bone replacement material of natural origin according to the prior art, and commercially available bovine bone substitution material TUTOBONE® (Tutogen Medical GmbH).
Figure 2 shows a SEM (Scanning Electron Microscopy) micrograph of the surface of a block of a hydrophilic dehydrated partially purified bone replacement material of equine origin containing 0.35 % w/w sorbitol and 1.4 % w/w of (HPO₄²⁻ and H₂PO₄⁻) according to the present invention, after incubation in DMEM (Dulbeco's Modified Eagle's Medium) containing 10 % FCS (Fetal Calf Serum).
Figure 3 schematically illustrates a bone block in accordance with one embodiment.
Figure 4 schematically illustrates a bone pin in accordance with one embodiment.
Figure 5 schematically illustrates a bone plate in accordance with one embodiment.
Figure 6 schematically illustrates a bone tapered key in accordance with one embodiment.
Figure 7 schematically illustrates a bone bar in accordance with one embodiment.
Figure 8 schematically illustrates bone granules in accordance with one embodiment.

### Detailed description of the invention

According to the present invention, by the term of "natural origin" is intended any bone replacement material, i.e. bone or bone-like material, that is derived from vertebrates including, but not limited to, all kinds of mammalians such as human, cow, horse, pig, etc. and any bone or bone-like material that is derived from invertebrates, such as corals. In certain embodiments, bovine, porcine or equine bone is used for the purpose of the present invention.

By the term "partially purified bone replacement material" is intended any bone replacement material from which substantially all organic non-collagenous material is removed while the inorganic, porous osseous structure and the collagenous structure are substantially preserved.

The term "hydrophilic" means that the dehydrated partially purified bone replacement material is capable of being wetted by water, therefore capable of being easily rehydrated by absorbing an isotonic solution or a physiological fluid, and thus presents no risk of forming an air bubble when implanted.

The term "saccharide" notably includes hexoses such as glucose, mannose or galactose; pentoses such as ribose or arabinose; ketoses such as ribulose or fructose; disaccharides such as sucrose, lactose, or maltose, oligosaccharides like cyclodextrin. The saccharide may be present in the D- or in the L- form. Certain embodiments utilize glucose.

Sugar alcohols included in the invention are notably sorbitol, maltitol, lactitol, mannitol and xylitol. Certain embodiments utilize sorbitol.

The term "phosphate group which is apt to react with the calcium ions of physiological fluids to give a precursor of hydroxyapatite" means any phosphate group which in the human body by reaction with the calcium ions of physiological fluids, in particular blood, preferably at a pH of 6.0 to 8.0 and a temperature of 36 to 38 °C, is prone to give a precursor of hydroxyapatite Ca₁₀(PO₄)₆(OH)₂, the thermodynamically most stable and less soluble of calcium phosphate salts which is an essential component of bones.

Sodium phosphate buffer is used as a physiologically acceptable salt, the phosphate groups apt to react with the calcium ions of physiological fluids apt to give a precursor of hydroxyapatite being then HPO₄²⁻ and H₂PO₄⁻.

The phosphate group contained in that material, which is apt to react with the calcium ions of physiological fluids to give a precursor of hydroxyapatite and is preferably selected from the group consisting of phosphate HPO₄²⁻ and H₂PO₄⁻, plays an important role in promoting bone formation.

It was shown in vitro that after incubation of a block of the hydrophilic dehydrated partially purified bone replacement material of the invention for 72 hours in a medium similar to physiological fluids which contained calcium ions, namely DMEM (Dulbeco's Modified Eagle's Medium) containing 10 % FCS (Fetal Calf Serum), the phosphate group reacts with the calcium ions to form of a white precipitate of calcium phosphates on collagen fibrils which was visible by SEM analysis (see Fig. 2). That precipitate was shown to contain two types of calcium phosphates of different morphologies, both of which can be described as precursors of hydroxyapatite.

Those in vitro experiments (see Example 4) provide a strong indication that in vivo, as in natural systems (see S. Gajjeraman et al. 2007 The Journal of Biological Chemistry 282, 2, pp. 1193-1204 and S. Bodhak et al. 2009 Acta Biomaterialia 5, pp. 2178-2188), the nucleation and growth of bone mineral products will take place on the collagen matrix of the hydrophilic dehydrated partially purified bone replacement material, the phosphate group contained in the latter which is released in physiological fluids playing a role in such reactions. With its low content of non physiological substance, the hydrophilic dehydrated partially purified bone replacement material of the invention will thus when implanted be in a position to mimic natural systems, the phosphate released from the hydrophilic dehydrated partially purified bone replacement material providing an initial boost to bone formation by starting the nucleation and growth process of mineral products.

The invention also concerns a hydrophilic dehydrated partially purified bone replacement material as defined above, which when incubated in a medium similar to physiological fluids containing calcium ions involves a reaction of the phosphate group with those calcium ions to give a precipitate of calcium phosphates on the collagen matrix, those calcium phosphates having the features of precursors of hydroxyapatite.

Upon implantation in various animals, notably rats and dogs, the hydrophilic dehydrated partially purified bone replacement material of the invention showed excellent new bone formation capacity with no or very few adverse histopathological events (inflammatory or foreign body reactions).

This new bone formation capacity is in line with the teachings of notably Chai YC et al., 2011, Tissue Engineering Part A, 17, 1083-97 "Probing the osteoconductive effect of calcium phosphate by using an in vitro biomimetic model". Those authors notably showed that phosphate calcium drives mesenchymial stem cells into the osteoblast lineage, thus presumably improving bone formation. Moreover Sogo Y et al., 2007, 2, 116-23 teach the coprecipitation of calcium phosphate with collagen in supersaturated calcium phosphate solution and its effect on mesenchymal stem cells, namely enhancing osteogenic differentiation.

The following has been unexpectedly found by the present inventors:
To be in a position to play a role in promoting bone formation, the phosphate group should be contained in a sufficient quantity, usually at least 0.7 w/w %, preferably at least 1.0 w/w %, of the hydrophilic dehydrated partially purified bone replacement material.

When released in excess in physiological fluids the phosphate group might cause large precipitation of calcium phosphates, leading to a possible adverse inflammatory response. Such a response does not occur when the phosphate group is not higher than 5.6 w/w %, preferably 2.8 w/w % of the hydrophilic dehydrated partially purified bone replacement material.

The hydrophilic dehydrated partially purified bone replacement material of the invention thus contains 0.7 to 5.6 w/w %, preferably 1.0 to 2.8 w/w %, of a phosphate group which is apt to react with the calcium ions of physiological fluids to give a precursor of hydroxyapatite.

The invention also concerns the use of 0.7 to 5.6 w/w % of a phosphate group apt to react with the calcium ions of physiological fluids to give a precursor of hydroxyapatite, selected from the group consisting of phosphate HPO₄²⁻ and H₂PO₄, for enhancing the new bone formation capacity of a partially purified bone replacement material of natural origin, wherein substantially all non-collagenous organic material is removed while inorganic, porous osseous structure and collagenous structure of natural bone are substantially preserved, with no or very few adverse histopathological events (inflammatory or foreign body reactions).

In certain embodiments, the hydrophilic dehydrated partially purified bone replacement material is coated with glucose and sodium phosphate buffer.

In certain embodiments, the hydrophilic dehydrated partially purified bone replacement material is coated with sorbitol and sodium phosphate buffer.

In certain embodiments, the hydrophilic dehydrated partially purified bone replacement material further contains one or more pharmaceutically active agents.

Examples of suitable pharmaceutically active agents are taurolidine, viricides, microbicides, antibiotics, amino acids, peptides, proteins, vitamins, co-factors for protein synthesis, hormones, living cells including stem cells, enzymes, antigenic agents, antitumor agents, immunosuppressants and growth factors.

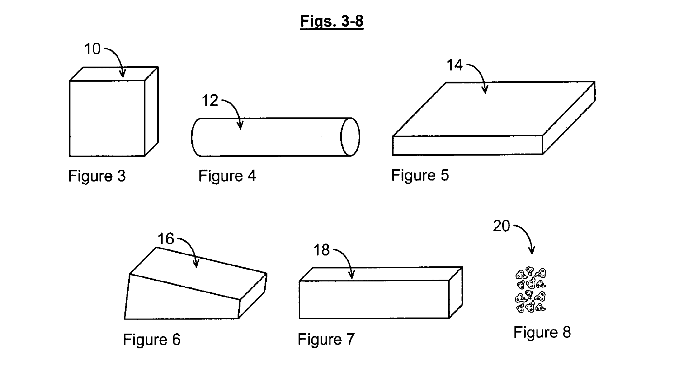

The dehydrated partially purified bone replacement material may have any shape including, without limitation, a block 10 (Fig. 3), a pin (bolt) 12 (Fig. 4), a plate 14 (Fig. 5), a tapered key or strip 16 (referred to as "cotter" in the U. K., Fig. 6), a bar 18 (Fig. 7), or a bone granulate 20 (Fig. 8), and the like.

In a first embodiment the structure is a block 10 as shown in Fig. 3, in a second embodiment a strip as shown in Fig.6, and in a third embodiment a granulate as shown in Fig. 8.

The invention further relates to the use of a bone replacement material as described herein as remodelling implant.

The invention also relates to the use of 0.05 to 1.5, preferably 0.05 to 0.8, in particular 0.05 to 0.5 w/w % of at least one of a saccharide or a sugar alcohol to confer hydrophilic properties to a partially purified bone replacement material of natural origin, wherein substantially all non-collagenous organic material is removed while inorganic, porous osseous structure and collagenous structure of natural bone are substantially preserved.

### Preparation of the hydrophilic dehydrated partially purified bone replacement material

The inventive hydrophilic dehydrated partially purified bone replacement material of natural origin may be produced by a process comprising:
(a) providing a partially purified bone replacement material of natural origin by first extracting lipids with organic solvents, there treating the delipidized material with a solution containing a chaotropic agent and carrying out extensive washing with water,
(b) soaking the partially purified bone replacement material of natural origin in a solution containing 0.05-0.85 w/w % of at least one of a saccharide or a sugar alcohol, thereby providing hydrophilicity to the partially purified bone replacement material, and 10-1000 mM of a phosphate group which is apt to react with the calcium ions of physiological fluids to give a precursor of this phosphate group being part of sodium phosphate buffer,
(c) freeze-drying the soaked hydrophilic partially purified bone replacement material, thereby performing dehydration, and
(d) sterilizing the hydrophilic dehydrated partially purified bone freeze-dried bone replacement material.

The organic solvents used for extracting lipids in step (a) may include methanol, ethanol, ethanol, propanol, hexane, cyclohexane, acetone and/or toluene.

Said chaotropic agent used in step (a) comprises, for example, urea, thiourea, guanidine HCl, guanidine thiocyanate, aminoguanidine HCl, aminoguanidine bicarbonate, guanidine carbonate, guanidine phosphate, or mixtures thereof.

In one embodiment the hydrophilic dehydrated partially purified bone replacement material of natural origin is produced by a process comprising the steps of:
(a) providing a partially purified bone replacement material of natural origin by first extracting lipids with organic solvents, there treating the delipidized material with a solution containing a chaotropic agent and carrying out extensive washing with water,
(b) soaking the dehydrated partially purified bone replacement material of natural origin in a solution containing 0.05-0.85 w/w % of at least one of a saccharide or a sugar alcohol, thereby providing hydrophilicity to the partially purified bone replacement material, and 10-1000 mM of a phosphate group selected from the group consisting of phosphate HPO₄²⁻ and H₂PO₄⁻, this phosphate group being part of sodium phosphate buffer,
(c) freeze-drying the soaked hydrophilic partially purified bone replacement material, thereby performing dehydration, and
(d) sterilizing the hydrophilic partially purified bone freeze-dried bone replacement material.

In one embodiment the solution used in step (b) contains 75-600 mM, in particular 100-200 mM, sodium phosphate buffer of pH 7.0.

The above process may also comprise a further intermediate dehydrating step performed on either on the delipidized bone replacement material of natural origin obtained during step (a), or the partially purified bone replacement material of natural origin obtained at the end of step (a), such as to disrupt the collagen structure and cause alteration of the orientation of the collagen fibers.

That intermediate dehydrating step is usually performed by vacuum-drying at a temperature above 0 °C, e.g. from 5 to 50 °C, in particular from 10 to 40 °C or by vigorously blowing an inert gas such as nitrogen though the material. Freeze-drying is not appropriate here because fixation of bone soft tissue by freezing results in the closed structure of natural bone remaining essentially the same without any significant disruption of the collagen structure.

By taking Scanning Electron Microscopy micrographs of the material before and after that dehydrating step, it can be shown that the material presents after that step a more open, disrupted and irregular structure of collagen than before, corresponding to an alteration of the orientation collagen fibers. That disruption of the collagen structure is particularly apparent when the intermediate dehydrating step is performed on the partially purified bone replacement material of natural origin obtained at the end of step (a).

Preferably the further intermediate dehydrating step is a vacuum-drying step performed at a temperature above 0 °C on the partially purified bone replacement material of natural origin obtained at the end of step (a), such as to disrupt the collagen structure and cause alteration of the orientation of the collagen fibers.

The invention further relates to a dehydrated partially purified bone replacement material prepared by the above process.

### Hydrophilicity and bone integration of the dehydrated partiallypurified bone replacement material

Bone integration, i.e., the direct physical connection of the implant material to the surrounding living host tissue may greatly increase the success of bone repair. Hydrophilicity is a major factor influencing bone integration in the case of bone replacement materials of animal origin. The more hydrophilic the material, the better and faster is the physical connection of the implant material to the surrounding tissue. There exists no generally accepted way to measure or quantify hydrophilicity and bone integration. An indirect measurement of bone integration is to detect the isotonic solution suction capacity of the graft.

The isotonic solution suction capacity of a dehydrated partially purified bone material is the property of that material to absorb an isotonic solution in its internal structure. That property depends on the pore structure, the wettability and the capillarity of the bone material and is a good indicator of bone integration, i.e. the direct physical connection of the implant material to the surrounding living host tissue, which plays an important role in the success of bone repair.

To measure the isotonic solution suction capacity of a block of solid bone replacement material, the following method was used: On a laboratory precision weighing balance, a defined amount of an isotonic PBS (Phosphate Buffer Saline) solution was placed in a dish. One block of a dehydrated bone replacement material of a defined weight was clamped in a height adjustable holding device beside the balance. The height of the device was then adjusted so that the bone replacement material block just touched the surface of the aqueous liquid in the dish. The height of the device was fixed so that the distance to the surface of the liquid remained the same throughout the measurement. In the block of hydrophilic bone replacement material, liquid was absorbed and the liquid uptake was detected with the balance as a function of time.

The isotonic solution suction capacity of a dehydrated partially purified bone replacement material is defined as the mass of isotonic solution absorbed. The relative isotonic suction capacity is defined as the isotonic solution suction capacity per mass unit of the dehydrated partially purified bone replacement material.

It has surprisingly been found that coating of dehydrated partially purified bone replacement material of natural origin with at least one of a saccharide or a sugar alcohol, and with a physiologically acceptable salt, so that it contains 0.05 to 1.5 w/w % of at least one of a saccharide or a sugar alcohol, enormously enhances the isotonic solution suction capacity.

The relative isotonic solution suction capacity of such treated bone replacement materials is at least 50 % within 5 minutes but may be at least 100 % within 5 minutes or even at least 200 % within 5 minutes.

In certain embodiments, it was found that about 50 % of the total liquid uptake is reached within less than about two minutes, or even within less than about one minute.

In certain embodiments, it was found that such a beneficial isotonic solution suction capacity can be achieved by coating the dehydrated partially purified bone replacement material of natural origin with a smaller amount of at least one of a saccharide or a sugar alcohol, so that it contains 0.05 to 0.8 w/w %, or even 0.05 to 0.5 w/w %, of at least one of a saccharide or a sugar alcohol.

These findings are associated with fundamental advantages, in particular:
- substantially immediate integration of the graft with the surrounding tissue;
- the surgeon does not need to pre-treat the bone replacement material prior to its use and therefore a decreased risk of contamination of the material may be obtained;
- the saccharide or sugar alcohol, which is a non physiological substance designed to be washed away by physiological fluids, is kept to a minimum, therefore the functioning of the human body is minimally disturbed and the implanted hydrophilic bone material will be optimally tolerated by the patient;
- improved and faster healing results than prior techniques.

The following examples illustrate the invention without restricting its scope.

### Example 1 Preparation of dehydrated partially purified blocks of spongiosa bone of equine origin containing 1.5 w/w % of glucose and 1.4 w/w % of HPO₄²⁻ and H₂PO₄⁻.

Spongiosa bone of equine origin was cut to blocks of 12 X 12 X 5 mm.

The following steps were performed on those blocks: selective washing steps with water and/or an organic solvent such as acetone or an alcohol (e.g. ethanol), virus deactivation steps using sodium hydroxide and/or hydrogen peroxide, delipidization with an organic solvent such as methanol, ethanol, propanol, hexane, cyclohexane, acetone, or toluene, and washing steps with a solution containing a chaotropic salt such as urea or guanidine hydrochloride at a temperature between 4°C to 35 °C followed. The resulting blocks were rinsed thoroughly with water and finally vacuum dried.

Observation of those blocks by Scanning Electron Microscopy showed that the native collagen structure was substantially preserved. Analysis of those blocks by various techniques including semiquantitative 2D SDS-PAGE showed the presence of less than 1 % of extractable proteins other than collagen.

The blocks were then soaked in a 0.86 w/w % glucose solution containing 200mM sodium sodium phosphate buffer of pH 7.0 (prepared by dissolving NaH₂PO₄ in demineralised water and adjusting the pH with sodium hydroxide) at room temperature for 3 hours, taken out of the aqueous solution with tweezers, then freeze dried and sterilized using gamma irradiation. The obtained blocks were shown to contain about 1.5 w/w % of glucose and 1.87 w/w % of HPO₄²⁻ and H₂PO₄⁻.

### Example 2 Preparation of dehydrated partially purified blocks of spongiosa bone of equine origin containing 0.35 w/w % of sorbitol and 1.4 w/w % of phosphate HPO₄²⁻ and H₂PO₄⁻

Spongiosa bone of equine origin was cut to blocks of 10 X 10 X 5 mm.

The following steps were performed on those blocks: selective washing steps with water and/or an organic solvent such as acetone or an alcohol (e.g. ethanol), virus deactivation steps using sodium hydroxide and/or hydrogen peroxide, delipidization with an organic solvent such as methanol, ethanol, propanol, hexane, cyclohexane, acetone, or toluene, and washing steps with a solution containing a chaotropic salt such as urea or guanidine hydrochloride at a temperature between 4°C to 35 °C followed. The resulting blocks were rinsed thoroughly with water and finally vacuum dried.

Observation of those blocks by Scanning Electron Microscopy showed that the native collagen structure was substantially preserved. Analysis of those blocks by various techniques including semiquantitative 2D SDS-PAGE showed the presence of less than 1 % of extractable proteins other than collagen.

The blocks were then soaked in a 0.2 w/w % sorbitol aqueous solution containing 150 mM sodium phosphate buffer of pH 7.0 (prepared by dissolving NaH₂PO₄ in demineralised water and adjusting the pH with sodium hydroxide) at room temperature for 2 hours, taken out of the aqueous solution with tweezers, then freeze-dried and sterilized using gamma irradiation. The obtained blocks were shown to contain about 0.35 w/w % of sorbitol and 1.4 w/w % of HPO₄²⁻ and H₂PO₄⁻.

### Example 2bis Preparation of "non-plasticized" dehydrated partially purified blocks of spongiosa bone of equine origin containing 0.35 w/w % of sorbitol and 1.4 w/w % of phosphate HPO₄²⁻ and H₂PO₄⁻

Spongiosa bone of equine origin was cut to blocks of 10 X 10 X 5 mm.

The following steps were performed on those blocks: selective washing steps with water and/or an organic solvent such as acetone or an alcohol (e.g. ethanol), virus deactivation steps using sodium hydroxide and/or hydrogen peroxide, delipidization with an organic solvent such as methanol, ethanol, propanol, hexane, cyclohexane, acetone, or toluene, and washing steps with a solution containing a chaotropic salt such as urea or guanidine hydrochloride at a temperature between 4°C to 35 °C followed. The resulting blocks were rinsed thoroughly with water, then submitted to a step of vacuum-drying at room temperature for 240 minutes at a pressure below 20'000 Pascal.

Observation of those blocks by Scanning Electron Microscopy before the step of vacuum-drying (dried by freeze-drying) and after that step showed a substantial difference in the collagen structure: After the vacuum-drying step the collagen structure was more open, disrupted and irregular, corresponding to an alteration of the orientation of collagen fibers.

Those blocks are thus "non-plasticized", i.e. do not correspond to the definition of "plasticized" in U.S. Patent NO. 6,293,970 and US2010/0030340 (see above page 3, lines 10-139.

Analysis of the blocks obtained after the vacuum-drying step by various techniques including semiquantitative 2D SDS-PAGE showed the presence of less than 1 % of extractable proteins other than collagen.

The blocks were then soaked in a 0.2 w/w % sorbitol aqueous solution containing 150 mM sodium phosphate buffer of pH 7.0 (prepared by dissolving NaH₂PO₄ in demineralised water and adjusting the pH with sodium hydroxide) at room temperature for 2 hours, taken out of the aqueous solution with tweezers, then freeze-dried and sterilized using gamma irradiation. The obtained blocks were shown to contain about 0.35 w/w % of sorbitol and 1.4 w/w % of HPO₄²⁻ and H₂PO₄⁻.

### Example 3 Measurement of isotonic solution suction capacity in correlation with time for dehydrated partially purified bone materials according to the invention

To measure the isotonic solution suction capacity of a block of solid bone replacement material, the following method was used:
On a laboratory precision weighing balance, a defined amount of an isotonic PBS solution was placed in a dish. One block of a dehydrated bone replacement material of a defined weight was clamped in a height adjustable holding device beside the balance. The height of the device was then adjusted so that the bone replacement material block just touched the surface of the aqueous liquid in the dish. The height of the device was fixed so that the distance to the surface of the liquid remained the same throughout the measurement. In the block of hydrophilic bone replacement material, liquid was absorbed and the liquid uptake was detected with the balance as a function of time.

The above method was used for measuring the isotonic solution absorption capacity of:
- 8 different blocks (having a density between 0.230 and 0.500 g/cm³) of a hydrophilic dehydrated partially purified bone replacement material of equine origin according to the invention, containing 0.35 w/w % of sorbitol and 1.4 w/w % of HPO₄²⁻and H₂PO₄⁻ prepared as described in Example 2
- 3 different blocks of a dehydrated partially purified bone replacement material of equine origin prepared as described in Example 2 second paragraph (without sorbitol or phosphate), and
- commercially available block of bovine substitution material TUTOBONE® (Tutogen Medical GmbH).

The results are represented in Figures 1A and 1B.

Figure 1A shows the isotonic solution suction capacity (liquid uptake in mg) in correlation with time for the above blocks.

Figure 1B shows the relative isotonic solution suction capacity (w/w ratio between the liquid uptake and the dehydrated block, expressed in %) in correlation with time for the above blocks.

As apparent from Figure 1A:
- the presence of 0.35 % sorbitol and 1.4 w/w % of HPO₄²⁻ and H₂PO₄⁻dramatically increases the isotonic solution suction capacity,
- more than 50 % of the total liquid uptake is reached within less than 2 minutes, or even less than 1 minute.

As apparent from Figure 1 B:
- the relative isotonic solution suction capacity with 5 minutes is at least 100 % for all blocks,
- but may be at least 150 % (5 blocks) or even at least 200 % (one block). The above experiments show the high hydrophilicity of the blocks of dehydrated partially purified bone materials according to the invention containing 0.35 w/w % of sorbitol and 1.4 w/w % of phosphate HPO₄²⁻ and H₂PO₄⁻.

In a series of similar experiments it was shown that blocks of dehydrated partially purified bone materials according to the invention containing 0.05, 0.2, 0.46, 1.14 or 1.50 w/w % of sorbitol and 1.4 w/w % of phosphate HPO₄²⁻ and H₂PO₄⁻ have the same high hydrophilicity (the measured relative isotonic solution absorption capacity was not significantly different for those various sorbitol contents).

### Example 4 Formation by reaction with calcium ions present in a medium similar to physiological fluids of a precipitate likely to be a precursor of hydroxyapatite on the surface of dehydrated partially purified bone materials according to the invention.

- A block of about 150 mg of a hydrophilic dehydrated partially purified bone replacement material of equine origin according to the invention, containing 0.35 w/w % of sorbitol and 1.4 w/w % of HPO₄²⁻ and H₂PO₄⁻prepared as described in Example 2, and
- 3 ml of 150 mM sodium phosphate buffer (containing the same amount of HPO₄²⁻ and H₂PO₄⁻ as the above block) without any block,
were incubated for 72 hours at 37 °C in 3 ml of in DMEM (Dulbeco's Modified Eagle's Medium) containing 10 % FCS (Fetal Calf Serum) (a medium similar to physiological fluids containing a concentration of calcium ions of about 65 mg/l).

The medium obtained by incubating the sodium phosphate buffer became and stayed turbid during all the incubation time whereas the medium obtained by incubating each the above block stayed clear during all the incubation time.

The above block was taken out of the incubation medium, fixed with 4% PFA (paraformaldehyde), dried and analysed by SEM (Scanning Electron Microscopy).

See Figure 2, which represents a micrograph of that block: The precipitate is depicted in white on the surface of the material, the collagen fibrils being visible as a greyish network underneath the precipitate.

To further characterize the reaction of the bloc with calcium ions present in the medium the Ca concentration of the medium was measured prior to and after 24h incubation with the bloc. The amount of calcium ions in solution after 24 hours incubation was measured to be much lower (about 2.0 mg/ml) than the initial amount of calcium ions (about 50 mg/ml), demonstrating reaction of the calcium ions with the phosphate groups

Since it was not possible to remove the precipitate from those blocks to analyse its composition, the precipitate formed from the turbid medium under similar conditions by incubating 3 ml of 150 mM sodium phosphate buffer without any block was washed, dried and analyzed via Scanning Electron Microscopy (REM) and Energy Dispersive X ray Spectroscopy (EDX). It was found that the precipitate contained calcium, phosphate and oxygen and included two types of calcium phosphates of different morphologies, one crystalline (sharp edged lamellas) with a molar ratio of calcium:phosphate 1:1 and the other cryptocrystalline or amorphous (fine particles agglomerates) with a ratio of 4:3. Both structural types represent a Ca:P molar ratio unequal to hydroxyapatite, which indicates that both phases can be described as precursors of hydroxyapatite. Indeed all calcium orthophosphate based salts should be transformed into hydroxyapatite, the thermodynamically most stable form of all calcium ortho-phosphate salts in an aqueous medium of pH of about 7 at a temperature of about 37°C, normal conditions found within the human body.

Those experiments show that in the hydrophilic dehydrated partially purified bone replacement material according to the invention, which contains a substantially lower amount of wetting agent than known hydrophilic bone replacement materials of the prior art, the phosphate groups HPO₄²⁻ and H₂PO₄⁻ react with the calcium ions of body fluids to form a precipitate on the surface of the biomaterial, this precipitate having the features of a precursor of hydroxyapatite.

The above in vitro experiments provide a strong indication that in vivo, as in natural systems, the nucleation and growth of bone mineral products will take place on the collagen matrix, the phosphate groups released in physiological fluids playing a role in such reactions.

## Claims

1. A hydrophilic dehydrated partially purified bone replacement material of natural origin, wherein substantially all non-collagenous organic material is removed while inorganic, porous osseous structure and collagenous structure of natural bone are substantially preserved, **characterized in that** the bone replacement material contains 0.05 to 1.5 w/w % of at least one of a saccharide or a sugar alcohol, and 0.7 to 5.6 w/w % of a phosphate group selected from the group consisting of phosphate HPO₄²⁻ and H₂PO₄⁻, this phosphate group being part of a physiologically acceptable salt which is sodium phosphate buffer.

2. A hydrophilic dehydrated partially purified bone replacement material according to claim 1, which contains 1.0 to 2.8 w/w % of a phosphate group selected from the group consisting of phosphate HPO₄²⁻ and H₂PO₄⁻.

3. A hydrophilic dehydrated partially purified bone replacement material according to according to any of claims 1 to 2, wherein the sugar alcohol is sorbitol.

4. A hydrophilic dehydrated partially purified bone replacement material according to any of claims 1 to 4, wherein the saccharide is glucose.

5. A hydrophilic dehydrated partially purified bone replacement material according to any of claims 1 to 4, **characterized in that** it is of bovine, porcine or equine origin.

6. A hydrophilic dehydrated partially purified bone replacement material according to any of claims 1 to 5 **characterized in that** it further contains a pharmaceutically active agent.

7. A hydrophilic dehydrated partially purified bone replacement material according to any of claims 1 to 6, which is in a form of a block.

8. A hydrophilic dehydrated partially purified bone replacement material according to any of claims 1 to 7, which when incubated in a medium similar to physiological fluids containing calcium ions involves a reaction of the phosphate group with those calcium ions to give a precipitate of calcium phosphates on the collagen matrix, those calcium phosphates having the features of precursors of hydroxyapatite.

9. A process for preparing a hydrophilic dehydrated partially purified bone replacement material according to any of claims 1 to 8 comprising the steps of:
(a) providing a partially purified bone replacement material of natural origin by first extracting lipids with organic solvents, there treating the delipidized material with a solution containing a chaotropic agent and carrying out extensive washing with water,
(b) soaking the dehydrated partially purified bone replacement material of natural origin in a solution containing 0.05-0.85 w/w % of at least one of a saccharide or a sugar alcohol, thereby providing hydrophilicity to the partially purified bone replacement material, and 10-1000 mM of a phosphate group selected from the group consisting of phosphate HPO₄²⁻ and H₂PO₄⁻, this phosphate group being part of sodium phosphate buffer,
(c) freeze-drying the soaked hydrophilic partially purified bone replacement material, thereby performing dehydration, and
(d) sterilizing the hydrophilic partially purified bone freeze-dried bone replacement material.

10. A process according to claim 9, wherein the solution used in step (b) contains 75-600 mM, preferably 100-200 mM, sodium phosphate buffer of pH 7.0.

11. A process according to claim 9 or 10, which comprises a further intermediate dehydrating step performed on the partially purified bone replacement material of natural origin obtained at the end of step (a), such as to disrupt the collagen structure and cause alteration of the orientation of the collagen fibers.

12. Use of 0.7 to 5.6 w/w % of a phosphate group apt to react with the calcium ions of physiological fluids to give a precursor of hydroxyapatite selected from the group consisting of phosphate HPO₄²⁻ and H₂PO₄, this phosphate group being part of sodium phosphate buffer, for enhancing the new bone formation capacity of a partially purified bone replacement material of natural origin from which substantially all non-collagenous organic material is removed while inorganic, porous osseous structure and collagenous structure of natural bone are substantially preserved, which contains 0.05 to 1.5 w/w % of at least one of a saccharide or a sugar alcohol.

## Patentansprüche

1. Hydrophiles dehydriertes, teilweise gereinigtes Knochenersatzmaterial natürlichen Ursprungs, wobei im Wesentlichen alles nicht-kollagene, organische Material entfernt ist, während eine anorganische, poröse, knöcherne Struktur und eine kollagene Struktur eines natürlichen Knochens im Wesentlichen erhalten geblieben sind, **dadurch gekennzeichnet, dass** das Knochenersatzmaterial 0,05 bis 1,5 w/w % von einem Saccharid und/oder einem Zuckeralkohol und 0,7 bis 5,6 w/w % von einer Phosphatgruppe enthält, die ausgewählt ist aus der Gruppe, die aus dem Phosphat HPO₄²⁻ und H₂PO₄⁻ besteht, wobei diese Phosphatgruppe Teil eines physiologisch annehmbaren Salzes ist, das ein Natriumphosphatpuffer ist.

2. Hydrophiles dehydriertes, teilweise gereinigtes Knochenersatzmaterial nach Anspruch 1, das 1,0 bis 2,8 w/w % von einer Phosphatgruppe enthält, die ausgewählt ist aus der Gruppe, die aus dem Phosphat HPO₄²⁻ und H₂PO₄⁻ besteht.

3. Hydrophiles dehydriertes, teilweise gereinigtes Knochenersatzmaterial nach einem der Ansprüche 1 bis 2, wobei der Zuckeralkohol ein Sorbitol ist.

4. Hydrophiles dehydriertes, teilweise gereinigtes Knochenersatzmaterial nach einem der Ansprüche 1 bis 3, wobei das Saccharid Glucose ist.

5. Hydrophiles dehydriertes, teilweise gereinigtes Knochenersatzmaterial nach einem der Ansprüche 1 bis 4, **dadurch gekennzeichnet, dass** es seinen Ursprung vom Rind, vom Schwein oder vom Pferd hat.

6. Hydrophiles dehydriertes, teilweise gereinigtes Knochenersatzmaterial nach einem der Ansprüche 1 bis 5, **dadurch gekennzeichnet, dass** es ferner einen pharmazeutisch aktiven Wirkstoff enthält.

7. Hydrophiles dehydriertes, teilweise gereinigtes Knochenersatzmaterial nach einem der Ansprüche 1 bis 6, das in der Form eines Blocks ist.

8. Hydrophiles dehydriertes, teilweise gereinigtes Knochenersatzmaterial nach einem der Ansprüche 1 bis 7, das dann, wenn es in einem Medium, das physiologischen Fluiden, die Calciumionen enthalten, ähnlich ist, inkubiert, eine Reaktion der Phosphatgruppe mit diesen Calciumionen auslöst, um eine Ausfällung von Calciumphosphaten auf der Kollagenmatrix zu ergeben, wobei die Calciumphosphate die Merkmale von Vorläufern von Hydroxyapatit aufweisen.

9. Verfahren zum Herstellen eines hydrophilen dehydrierten, teilweise gereinigten Knochenersatzmaterials nach einem der Ansprüche 1 bis 8, wobei das Verfahren die folgenden Schritte umfasst:
(a) Bereitstellen eines teilweise gereinigten Knochenersatzmaterials natürlichen Ursprungs durch erstens ein Extrahieren von Lipiden mit organischen Lösemitteln, dann Behandeln des so entlipidisierten Materials mit einer Lösung, die ein chaotropes Mittel enthält, und dann Ausführen eines ausgedehnten Waschens mit Wasser,
(b) Einweichen des dehydrierten, teilweise gereinigten Knochenersatzmaterials natürlichen Ursprungs in einer Lösung, die 0,05 bis 0,85 w/w % von einem Saccharid und/oder einem Zuckeralkohol enthält, wodurch das teilweise gereinigte Knochenersatzmaterial mit einer hydrophilen Eigenschaft versehen wird, und 10 bis 1.000 mM einer Phosphatgruppe enthält, die ausgewählt ist aus der Gruppe, die aus dem Phosphat HPO₄²⁻ und H₂PO₄⁻ besteht, wobei diese Phosphatgruppe Teil eines Natriumphosphatpuffers ist,
(c) Gefriertrocknen des eingeweichten hydrophilen, teilweise gereinigten Knochenersatzmaterials, wodurch eine Dehydration durchgeführt wird, und
(d) Sterilisieren des hydrophilen, teilweise gereinigten, gefriergetrockneten Knochenersatzmaterials.

10. Verfahren nach Anspruch 9, wobei die in Schritt (b) verwendete Lösung 75 bis 600 mM, vorzugsweise 100 bis 200 mM, Natriumphosphatpuffer von pH 7,0 enthält.

11. Verfahren nach Anspruch 9 oder 10, das einen weiteren Dehydrierungs-Zwischenschritt umfasst, der auf dem teilweise gereinigten Knochenersatzmaterial natürlichen Ursprungs, das am Ende von Schritt (a) erhalten worden ist, durchgeführt wird, um so die Kollagenstruktur zu spalten und um eine Änderung der Orientierung der Kollagenfasern zu veranlassen.

12. Verwendung von 0,7 bis 5,6 w/w % einer Phosphatgruppe, die geeignet ist, um mit den Calciumionen von physiologischen Fluiden zu reagieren, um einen Vorläufer von Hydroxyapatit zu ergeben, ausgewählt aus der Gruppe, die aus HPO₄²⁻ und H₂PO₄⁻ besteht, wobei diese Phosphatgruppe Teil eines Natriumphosphatpuffers ist, um die neue Knochenbildungskapazität eines teilweise gereinigten Knochenersatzmaterials natürlichen Ursprungs zu verbessern, aus dem im Wesentlichen alles nicht-kollagene, organische Material entfernt ist, während eine anorganische, poröse, knöcherne Struktur und eine kollagene Struktur eines natürlichen Knochens im Wesentlichen erhalten geblieben sind, wobei das Knochenersatzmaterial 0,05 bis 1,5 w/w % von einem Saccharid und/oder einem Zuckeralkohol enthält.

## Revendications

1. Matière hydrophile déshydratée partiellement purifiée d'origine naturelle pour remplacement d'os, dans laquelle sensiblement toute la matière organique non collagène est enlevée tandis que la structure osseuse poreuse inorganique et la structure collagène de l'os naturel sont sensiblement conservées, **caractérisée en ce que** la matière de remplacement d'os contient 0,05 à 1,5 % M/M d'au moins un d'un saccharide ou d'un alcool glucidique, et 0,7 à 5,6 % M/M d'un groupe phosphate sélectionné à partir du groupe constitué par un phosphate HPO₄²⁻ et H₂PO₄⁻, ce groupe phosphate faisant partie d'un sel acceptable d'un point de vue physiologique qui est un tampon de phosphate de sodium.

2. Matière hydrophile déshydratée partiellement purifiée pour remplacement d'os selon la revendication 1, qui contient 1,0 à 2,8 % M/M d'un groupe phosphate sélectionné à partir du groupe consistant en un phosphate HPO₄2⁻ et H₂PO₄⁻.

3. Matière hydrophile déshydratée partiellement purifiée pour remplacement d'os selon n'importe laquelle des revendications 1 à 2, dans laquelle l'alcool glucidique est du sorbitol.

4. Matière hydrophile déshydratée partiellement purifiée pour remplacement d'os selon n'importe laquelle des revendications 1 à 4, dans laquelle le saccharide est du glucose.

5. Matière hydrophile déshydratée partiellement purifiée pour remplacement d'os selon n'importe laquelle des revendications 1 à 4, **caractérisée en ce qu'**elle est d'origine bovine, porcine ou chevaline.

6. Matière hydrophile déshydratée partiellement purifiée pour remplacement d'os selon n'importe laquelle des revendications 1 à 5, **caractérisée en ce qu'**elle contient en outre un agent actif d'un point de vue pharmaceutique.

7. Matière hydrophile déshydratée partiellement purifiée pour remplacement d'os selon n'importe laquelle des revendications 1 à 6, qui est en forme d'un bloc.

8. Matière hydrophile déshydratée partiellement purifiée pour remplacement d'os selon n'importe laquelle des revendications 1 à 7, qui lorsqu'elle est incubée dans un milieu similaire à des fluides physiologiques contenant des ions de calcium implique une réaction du groupe phosphate avec ces ions de calcium pour donner à un précipité de phosphates de calcium sur la matrice de collagène, ces phosphates de calcium ayant les particularités de précurseurs d'hydroxyapatite.

9. Procédé pour préparer une matière hydrophile déshydratée partiellement purifiée pour remplacement d'os selon n'importe laquelle des revendications 1 à 8, comprenant les étapes de :
(a) fourniture d'une matière de remplacement d'os partiellement purifiée d'origine naturelle en extrayant d'abord des lipides avec des solvants organiques, en traitant ensuite la matière délipidée avec une solution contenant un agent chaotropique et en effectuant un lavage intensif avec de l'eau,
(b) imbibition de la matière déshydratée partiellement purifiée d'origine naturelle pour remplacement d'os dans une solution contenant 0,05 à 0,85 % M/M d'au moins un d'un saccharide ou d'un alcool glucidique, procurant ainsi un caractère hydrophile à la matière partiellement purifiée pour remplacement d'os, et 10 à 1 000 mM d'un groupe phosphate sélectionné à partir du groupe constitué par un phosphate HPO₄2⁻ et H₂PO₄⁻, ce groupe phosphate faisant partie d'un tampon de phosphate de sodium,
(c) lyophilisation de la matière hydrophile partiellement purifiée pour remplacement d'os imbibée, effectuant ainsi une déshydratation, et
(d) stérilisation de la matière hydrophile partiellement purifiée lyophilisée osseuse pour remplacement d'os.

10. Procédé selon la revendication 9, dans lequel la solution utilisée dans l'étape (b) contient 75 à 600 mM, de préférence 100 à 200 mM, d'un tampon de phosphate de sodium de pH 7,0.

11. Procédé selon la revendication 9 ou 10, qui comprend une étape de déshydratation intermédiaire supplémentaire effectuée sur la matière partiellement purifiée d'origine naturelle pour remplacement d'os obtenue à la fin de l'étape (a), de façon à perturber la structure de collagène et provoquer une modification de l'orientation des fibres de collagène.

12. Utilisation de 0,7 à 5,6 % M/M d'un groupe phosphate apte à réagir avec les ions de calcium de fluides physiologiques pour donner un précurseur d'hydroxyapatite sélectionné à partir du groupe constitué par du phosphate HPO₄2⁻ et H₂PO₄⁻, ce groupe phosphate faisant partie d'un tampon de phosphate de sodium, pour améliorer la capacité de formation de nouvel os d'une matière partiellement purifiée d'origine naturelle pour remplacement d'os à partir de laquelle sensiblement toute la matière organique non collagène est enlevée tandis que la structure osseuse poreuse inorganique et la structure collagène de l'os naturel sont sensiblement conservées, qui contient 0,05 à 1,5 % M/M d'au moins un d'un saccharide ou d'un alcool glucidique.
